# EUROPÄISCHE PATENTSCHRIFT

(11) **EP 3 419 529 B1**
(45) Veröffentlichungstag und Bekanntmachung des Hinweises auf die Patenterteilung: **25.11.2020**
(21) Anmeldenummer: 17797891.3
(22) Anmeldetag: 27.10.2017
(51) Int. Cl.: A61B 17/132

(54) **ABSCHNÜRVORRICHTUNG FÜR KÖRPERTEILE**
CONSTRICTION DEVICE FOR BODY PARTS
DISPOSITIF DE LIGATURE POUR PARTIES DU CORPS

(30) Priorität: 14.11.2016 DE 202016106370 U
(43) Veröffentlichungstag der Anmeldung: 02.01.2019
(73) Patentinhaber: Kimetec GmbH, 71254 Ditzingen (DE)
(72) Erfinder: IHLE, Caroline, 71706 Markgröningen (DE); KIRCHNER, Claudia, 71706 Markgröningen (DE); KIRCHNER, Hansjörg, 71706 Markgröningen (DE)
(74) Vertreter: Fleck, Hermann-Josef
(86) Internationale Anmeldenummer: PCT/EP2017/077643
(87) Internationale Veröffentlichungsnummer: WO 2018/086916

(56) Entgegenhaltungen:
- JP-Y1- 3 114 890
- KR-B1- 101 559 761
- US-A- 2 754 825
- US-A1- 2005 273 134

## Beschreibung

Die Erfindung bezieht sich auf eine Abschnürvorrichtung für Körperteile, insbesondere Venenstauer, mit einem um ein Körperteil legbaren flexiblen Abschnürband mit einer Verschlussvorrichtung, mittels deren das Abschnürband in seinem als geschlossene Schlaufe um das Körperteil gelegten Zustand verriegelbar ist.

Eine Abschnürvorrichtung für Körperteile dieser Art ist in der US 2005/273134 A1 angegeben. Bei dieser bekannten Abschnürvorrichtung ist ein flexibles Abschnürband mit einer Verschlussvorrichtung und einer davon separaten Spanneinheit versehen. Die Spanneinheit weist ein stabförmiges Spannteil auf, das mit einem Schlitz zum Durchführen des Abschnürbands versehen ist und durch Drehen eine Spannwirkung in dem um ein Körperteil gelegten Abschnürband bewirkt. Das Spannteil kann in einem bestimmten Spannungszustand mittels eines Fixierbands an dem Abschnürband festgelegt werden.

Bei einer in der KR 101 559 761 B1 gezeigten Abschnürvorrichtung für Körperteile wird ein flexibles Abschnürband, das eine Verschlussvorrichtung und eine davon separate Spanneinheit aufweist, mittels eines um eine vertikale Achse drehbaren Spannknopfes gespannt, welcher mit einem Zahnabschnitt in eine lineare Zahnstruktur eingreift.

Eine in der US 2 754 825 A gezeigte Abschürvorrichtung für Körperteile weist zum Spannen eines flexiblen Abschnürbandes, welches mit einer separaten Verschlussvorrichtung versehen ist, eine vertikal zur Oberfläche eines abzuschnürenden Körperteils gerichtete Gewindestange auf, die sich an einem Widerlager abstützt.

In der JP 3 114890 Y1 ist eine Abschnürvorrichtung für Körperteile dargestellt, die eine von einer Verschlussvorrichtung separate Spanneinheit mit einer Gehäuseeinheit aufweist, welche sich auf der Oberfläche des abzuschnürenden Körperteils abstützt und durch die ein um das Körperteil legbares flexibles Abschnürband geführt ist.

Eine weitere Abschnürvorrichtung ist in der WO 2015/070992 A1 angegeben. Bei dieser bekannten, als Venenstauer ausgebildeten Abschnürvorrichtung besteht das Abschnürband aus dünnem, flexiblem Fasermaterial, wie Gewebe oder insbesondere Vlies oder auch Filz oder papierartigem Material und ist reißfest mit hoher Zugfestigkeit und vorzugsweise flauschig ausgebildet. Das Vlies kann z. B. unter Wasserstrahlverfestigung hergestellt sein, womit auch relativ schmale, dünne Vliesbänder eine genügend hohe Festigkeit für die Abschnürung erhalten. Das sehr flexible Abschnürband ist auch mit einer Verschlussvorrichtung, und zwar in besonders vorteilhafter Weise mit einem Klettverschluss, versehen und kann bei einfacher Handhabung zu einer geschlossenen Schlaufe um den betreffenden Körperteil, wie Arm oder Bein, gelegt und unter bestimmter Spannung mittels der Verschlussvorrichtung verriegelt werden. In dem Abschnürband kann weiterhin vorteilhaft auch ein sogenannter Spannungsindikator integriert sein, das heißt ein elastischer Abschnitt, der sich beim Anlegen des Abschnürbandes um das Körperteil elastisch dehnen kann und für den Anwender der Abschnürvorrichtung eine Kontrollmöglichkeit eines gewissen Spannzustandes bietet. Bei Ausbildung der Verschlussvorrichtung als Klettverschluss ist in vorteilhafter Ausgestaltung das Abschnürband selbst aus Material mit Schlaufen als einem ersten Verschlussteil ausgebildet und mit einem Hakenelemente tragenden Abschnitt versehen, der den zweiten Verschlussteil bildet. Die Abschnürvorrichtung ist bei diesem Aufbau besonders einfach ausgebildet, leicht handhabbar und besonders vorteilhaft für den hygienischen Ansprüchen gerecht werdenden Einmalgebrauch geeignet.

Eine ähnliche Abschnürvorrichtung wie in der WO 2015/070992 A1 ist auch in der DE 20 2014 105 480 U1 gezeigt.

Zwar kann bei den genannten Abschnürvorrichtungen der Spannungszustand der um das Körperteil gelegten Schlaufe durch entsprechenden Zug beim Anlegen des Abschnürbandes durch den Benutzer variiert und über den Spannungsindikator kontrolliert werden, jedoch können sich Situationen ergeben, in denen der Spannungszustand anders eingestellt bzw. nachreguliert werden sollte. Dies könnte durch Öffnen der Verschlussvorrichtung und Wiederverschließen geschehen.

Bei einer in der DE 818 226 B vorgeschlagenen Vorrichtung zum Abschnüren von Körperteilen ist eine Spannvorrichtung mit einer verdrehbaren Rolle oder Walze versehen, welche mit einem selbsthemmenden Schneckentrieb zusammenarbeitet, der seitlich an einem Gestellteil angeordnet und mit einer Handhabe versehen ist. Ungünstig ist hierbei ein Schmierteil für die Schneckenradmechanik und auch der Aufbau und die Handhabung mit dem Einfädelmechanismus erscheinen aufwändig.

Eine in der US 2010/0057120 A1 gezeigte Abschnürvorrichtung weist eine unhandliche Knebeleinheit zum Spannen auf, die für eine Feinjustierung weniger geeignet ist.

Bei einer in der US 35 038 A gezeigten Abschnürvorrichtung ist eine für die Handhabung ungünstige Einfädeleinheit vorhanden, wobei ein Rast- und Betätigungsmechanismus seitlich angeordnet ist und sich ebenfalls Nachteile bei der Bedienung ergeben können.

Eine in der JPS31-14890Y1 gezeigte Abschnürvorrichtung besitzt eine relativ hoch auf der Oberfläche eines abzuschnürenden Körperteils aufbauende, und damit relativ schwer zu fixierende und zu stabilisierende Spannvorrichtung mit einer für die Bedienung nicht einfachen Banddurchführung und Verschlussvorrichtung.

Bekannt sind auch weitere Abschnürbänder mit einer gehäuseartigen Verschlussvorrichtung, die auch ein Nachregulieren des Spannzustandes der Schlaufe nach dem Anlegen ermöglicht, wie z. B. in der DE 10161749 C1 gezeigt. Jedoch ist ein solcher Aufbau relativ aufwendig und auch für den Einmalgebrauch kaum geeignet.

Der vorliegenden Erfindung liegt die Aufgabe zugrunde, eine Abschnürvorrichtung der eingangs genannten Art bereitzustellen, die bei möglichst einfachem, insbesondere auch Gesichtspunkte des Einmalgebrauchs erfüllendem Aufbau ein bedienerfreundliches Einstellen der Spannschlaufe ermöglicht.

Diese Aufgabe wird mit den Merkmalen des Anspruchs 1 gelöst. Hierbei ist vorgesehen, dass eine von der Verschlussvorrichtung separate Spanneinheit vorhanden ist, durch die ein innerhalb der Schlaufe liegender Abschnitt des Abschnürbands führbar ist und mittels deren die um das Körperteil gelegte, mittels der Verschlussvorrichtung verriegelte Schlaufe spannbar und in einem gewünschten Spannzustand mittels eines Arretiermechanismus arretierbar ist.

Bei der erfindugsgemäßen Abschnürvorrichtung ist weiterhin vorgesehen, dass die Spanneinheit ein auf einem Abschnitt des Abschnürbandes im Bereich der Schlaufe mit einer rutschfesten Unterseite aufsetzbares Unterteil aufweist. Die Spanneinheit kann hierbei einfach an einer geeigneten, gut zugänglichen Stelle des zu einer Schlaufe um das Körperteil gelegten Abschnürbandes aufgesetzt werden und wird dort unverrutschbar gehalten, wobei die Unterseite mit einer griffigen Struktur versehen ist, beispiels einer Aufrauhung oder gummiartigen Auflage oder Oberfläche, die mit der zugekehrten Oberfläche des Abschnürbandes griffig zusammenwirkt.

Die erfindungsgemäße Ausgestaltung besteht weiterhin darin, dass die Spanneinheit ein auf dem Unterteil drehbar gelagertes Spannteil aufweist, welches mit einem Schlitz zum Durchführen des Abschnürbandes versehen ist, und dass das Abschnürband durch Drehen des Spannteils relativ zu dem Unterteil spannbar ist, wobei das Spannteil gegen ein Drehen entgegen der Spannrichtung mittels des Arretiermechanismus blockiert ist. Das Spannen mittels des Spannteils wird besonders begünstigt, wenn das Abschnürband aus sehr flexiblem, reißfestem Material besteht, wie es auch in der eingangs genannten WO 2015/070992 A1 beschrieben ist.

Die weitere erfindungsgemäße Ausgestaltung besteht darin, dass der Arretiermechanismus einen an dem Spannteil angeordneten Arretierabschnitt und einen diesem zugekehrten und mit diesem zusammenwirkenden, an dem Unterteil angeordneten Arretiergegenabschnitt aufweist.

Die erfindungsgemäße Abschnürvorrichtung weist weiterhin das Merkmal auf dass das Spannteil um eine bezüglich einer Tangentialebene des Körperteils in einer Berührungslinie aufrecht stehende Drehachse drehbar an dem Unterteil in einem Drehlager gelagert ist.Durch den Aufbau der Abschnürvorrichtung mit der getrennten Spanneinheit lässt sich bei um das Körperteil gelegter und verriegelter Schlaufe der Spannzustand der Schlaufe gezielt einstellen bzw. nachregulieren und in dem gewünschten Spannzustand durch die Arretierung mittles des ebenfalls an der Spanneinheit vorhandenen Arretiermechanismus halten. Ist zudem die Spanneinheit als separate Einheit getrennt von dem Abschnürband ausgebildet und auf dieses aufsetzbar, so kann das Abschnürband getrennt von der Spanneinheit entsorgt und vorteilhaft für den Einmalgebrauch ausgebildet sein, beispielsweise wie in der eingangs genannten WO 2015/070992 A1, wobei ein dort gezeigter Spannungsindikator entfallen und das Abschnürband entsprechend einfach ausgebildet sein kann. Auch lässt durch eine gewisse Dehnbarkeit des am Körperteil angelegten Abschnürbandes, insbesondere wenn dieses eine gewisse Eigenelastizität besitzt, die von dem Benutzer bei Betätigung der Spanneinheit erzeugte Spannung bei lösbarem Arretiermechanismus z. T. wieder zurückstellen, so dass bei zu hoher Spannung auch ein definiertes Rückführen der Spannung in einen gewünschten geringeren Spannungszustand ermöglicht wird.

Eine für einen einfachen Aufbau und eine einfache Bedienung vorteilhafte Ausgestaltung besteht darin, dass die Verschlussvorrichtung als Klettverschluss, Druckknopfverschluss oder Hakenverschluss ausgebildet ist, wobei die Ausbildung mit einem Klettverschluss eine besonders einfache Ausgestaltung und Handhabung ermöglicht. Hierbei ist in besonders vorteilhafter Weise für den Aufbau und die Bedienung vorgesehen, dass die Verschlussvorrichtung als Klettverschluss mit zwei verriegelnd ineinandergreifenden Verschlussteilen ausgebildet ist.

Eine dabei weiterhin vorteilhafte Ausgestaltung besteht darin, dass das Abschnürband mit den ersten Verschlussteil bildenden Schlaufen einerseits und in diese zum Herstellen des Verschlusses verriegelnd eingreifenden, den zweiten Verschlussteil bildenden Hakenelementen andererseits versehen ist.

Weitere vorteilhafte Maßnahmen für den Aufbau und die Funktion bestehen darin, dass der erste Verschlussteil aus dem Bandmaterial selbst und der zweite Verschlussteil aus einem auf dem Bandmaterial befestigten Materialabschnitt gebildet ist.

Die Bedienung und der Aufbau werden ferner dadurch begünstigt, dass der Arretierabschnitt und der Arretiergegenabschnitt als um die Drehachse angeordnete komplementäre Zahnstrukturen mit in Spannrichtung flachen, aufeinandergleitenden Zahnflanken und entgegen der Spannrichtung steilen, blockierenden Zahnflanken versehen sind.

Eine für die Bedienung weiterhin vorteilhafte Ausgestaltung besteht darin, dass eine Freigabevorrichtung vorhanden ist, mittels deren der arretierte Spannzustand aufhebbar ist. Beispielsweise kann das Freigeben durch ein Anheben des Spannteils relativ zu dem Unterteil geschehen.

Ein vorteilhafter Aufbau bei zuverlässiger Funktion wird dabei dadurch erreicht, dass die Freigabevorrichtung zum Freigeben des arretierten Spannzustands durch Anheben des Spannteils entgegen einer dieses mit seinem Arretiergegenabschnitt in den Arretierabschnitt zwingenden Andruckkraft ausgebildet ist.

Der Aufbau und die Funktion werden dabei auch dadurch begünstigt, dass die Andruckkraft mittels des Abschnürbands in dessen Spannzustand erzeugt ist.

Eine für die Handhabung und den Aufbau weitere vorteilhafte Ausgestaltung ergibt sich dadurch, dass die Freigabevorrichtung in den Bereich zwischen dem Spannteil und dem Unterteil einschiebbare, mit Abschrägungen versehene verschiebbare Freigabemittel aufweist.

Für den Aufbau und die Montage ist auch die Ausgestaltung in der Weise vorteilhaft, dass das Spannteil mittels eines Fixierteils unverlierbar an dem Unterteil gehalten ist.

Eine für die Handhabung weitere vorteilhafte Ausgestaltung besteht darin, dass die Unterseite des Unterteils mit einem Befestigungsteil, insbesondere Klettverschlussteil, versehen ist, mittels dessen die Spanneinheit auf dem Abschnürband abnehmbar fixierbar ist. Durch den so ausgebildeten Befestigungsteil wird die auf das Abschnürband aufgesetzte Spanneinheit selbsttätig an diesem gehalten, wodurch die Handhabbarkeit vereinfacht wird. Beispielsweise kann der Befestigungsteil ein mit einem Schlaufenteil zusammenwirkender Hakenteil sein, wenn der Schlaufenteil an dem Abschnürband ausgebildet ist. Zum Entsorgen des Abschnürbandes kann die Spanneinheit einfach von diesem entfernt werden.

Die Erfindung wird nachfolgend anhand von Ausführungsbeispielen unter Bezugnahme auf die Zeichnungen näher erläutert. Es zeigen:
- Fig. 1: eine um ein Körperteil zu einer Schlaufe gelegte Abschnürvorrichtung mit einem Abschnürband und einer Spanneinheit in perspektivischer Ansicht,
- Fig. 2: einen Querschnitt der in Fig. 1 gezeigten Abschnürvorrichtung,
- Fig. 3: eine Spanneinheit der Abschnürvorrichtung in fünf verschiedenen Ansichten, nämlich von vorn (mittlere Darstellung), von oben (obere Darstellung), von unten (unter Darstellung), von der Seite (rechte Darstellung) und in einem Längsschnitt entlang einer Schnittlinie EE (linke Darstellung),
- Fig. 4: einen Unterteil und Freigabemittel der Spanneinheit in auseinandergezogener, perspektivischer Darstellung von schräg oben,
- Fig. 5: die Spanneinheit mit dem Unterteil, den Freigabemitteln, einem Spannteil und einem Fixierteil in auseinandergezogener, perspektivischer Darstellung von schräg unten,
- Fig. 6: den Unterteil mit aufgelegten Freigabemitteln und den davon entfernten Spannteil der Spanneinheit in perspektivischer Darstellung von schräg oben und
- Fig. 7: die Spanneinheit mit Unterteil, darauf aufgesetzten Freigabemitteln und aufgesetztem Spannteil sowie abgehobenem Fixierteil in perspektivischer Darstellung von schräg oben.

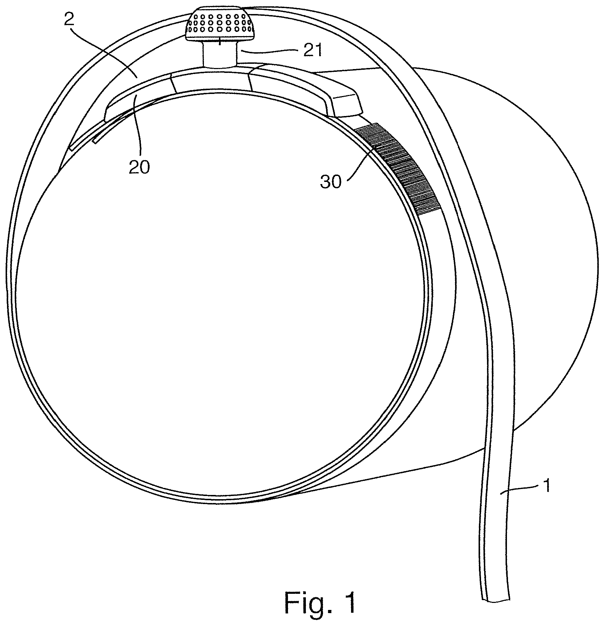

Fig. 1 zeigt eine Abschnürvorrichtung mit einem Abschnürband 1, welches um ein Körperteil, wie z. B. einen Arm eines Patienten zu einer Schlaufe gelegtes Abschnürband 1 sowie eine auf dem Abschnürband 1 aufgesetzte Spanneinheit 2 aufweist. Die Spanneinheit 2 ist dabei zwischen einem auf dem Körperteil 5 aufliegenden und einem von diesem abliegenden Abschnitt des Abschnürbandes 1 angeordnet, wobei der abliegende Abschnitt durch einen Spannteil 21 geführt ist. Zum Verriegeln der Schlaufe ist das Abschnürband 1 mit einer von der Spanneinheit 2 getrennten Verschlussvorrichtung versehen, die vorliegend als Klettverschluss ausgebildet ist und einen an oder in dem Abschnürband 1 angeordneten ersten Verschlussteil und einen an oder in dem Abschnürband 1 ausgebildeten zweiten Verschlussteil 30 aufweist. Wie aus Fig. 1 ersichtlich, ist die Spanneinheit 2 auf dem zwischen dem zweiten Verschlussteil 30 und dem Ende des auf dem Körperteil 5 aufliegenden Abschnitt der Schlaufe bzw. des Abschnürbandes 1 angeordnet, sodass die Spanneinheit 2 nach Verriegeln der Verschlussvorrichtung innerhalb der verriegelten Schlaufe liegt und der Spannzustand der Schlaufe mittels des Spannteils 21 veränderbar ist, indem das Spannteil 21 um seine bezüglich des Körperteils 5 vertikale Achse gedreht wird. Hierzu ist das Spannteil 21 auf einem Unterteil 20 drehbar gelagert, welches den dem körperteilseitigen Abschnitt der Schlaufe zugekehrten Teil der Spanneinheit 2 bildet.

Das Abschnürband 1 ist vorteilhaft aus einem dünnen, flexiblen und dabei reißfesten Material ausgebildet, wie es in der in der Beschreibungseinleitung genannten WO 2015/070992 A1 beschrieben ist. Durch die sehr flexible Ausbildung des Abschnürbandes 1 liegt dieses flach an dem Körperteil 5 an und kann leicht zum Durchführen durch das Spannteil 21 entsprechend verformt werden, ohne den flach an dem Körperteil 5 anliegenden Schlaufenbereich wesentlich zu verformen. Der durch das Spannteil 21 geführte Abschnitt des Abschnürbandes kann anschließend zum Verriegeln der Schlaufe mit seinem ersten Verschlussteil an dem zweiten Verschlussteil 30 in Verbindung gebracht werden.

Wie auch in der eingangs genannten WO 2015/070992 A1 näher ausgeführt, ist der hier erste Verschlussteil vorteilhaft über die gesamte oder zumindest einen Großteil der Länge des Abschnürbandes 1 ausgebildet. Hierzu ist vorzugsweise das Material des Abschnürbandes 1 selbst mit entsprechenden Verschlusselementen, insbesondere mit Schlaufenteilchen, für den Klettverschluss versehen. Der zweite Verschlussteil 30 ist beispielsweise als Hakenabschnitt auf dem Abschnürband 1 z. B. durch Verschweißen, Vernähen oder Verkleben angebracht oder ebenfalls in das Abschnürband 1 integriert. Vorteilhaft geeignetes Material des Abschnürbandes 1 ist ein dünnes Vliesmaterial, wobei das Vlies unter Wasserstrahlverfestigung hergestellt ist und eine sehr hohe Reißfestigkeit, bei dünner, dennoch flauschiger Ausbildung erhält, wie auch in der WO 2015/070992 A1 beschrieben. Das so ausgebildete Bandmaterial 1 kann z. B. aus flächigem Material in geeignet breite Streifen von z. B. zwischen 0,6 cm und 5 cm geschnitten und als lange, z. B. aufgerollte oder gefaltete Bänder zur Verfügung gestellt werden, die für die einzelnen Abschnürbänder 1 nach Bedarf abgelängt werden können. Die langen Bänder sind hierzu vorzugsweise in regelmäßigen Abständen mit den zweiten Verschlussteilen 30, insbesondere also Hakenabschnitten, versehen, sodass die betreffende Länge der Abschnürbänder 1 durch den Abstand der zweiten Verschlussteile 30 vorgegeben ist, wobei in geeignetem Abstand von den Verschlussteilen 30 Abrissstellen (z. B. durch Perforation oder Einschnitte oder Einkerbungen) vorgesehen sein können. Die Breite der Abschnürbänder 1 kann sich nach dem Verwendungszweck, beispielsweise als Venenstauer für Erwachsene (breitere Bänder) oder Kinder bzw. Kleinkinder (schmale Bänder), richten. In jedem Falle sind die so ausgebildeten Abschnürbänder 1 einfach herzustellen, einfach und für den Patienten angenehm zu handhaben und ohne große Belastung für die Umwelt entsorgbar, womit auch hohe Hygieneanforderungen erfüllt sind.

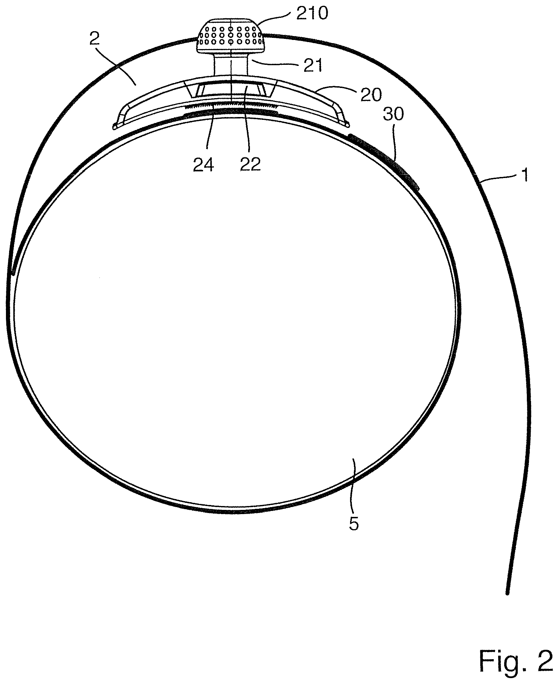

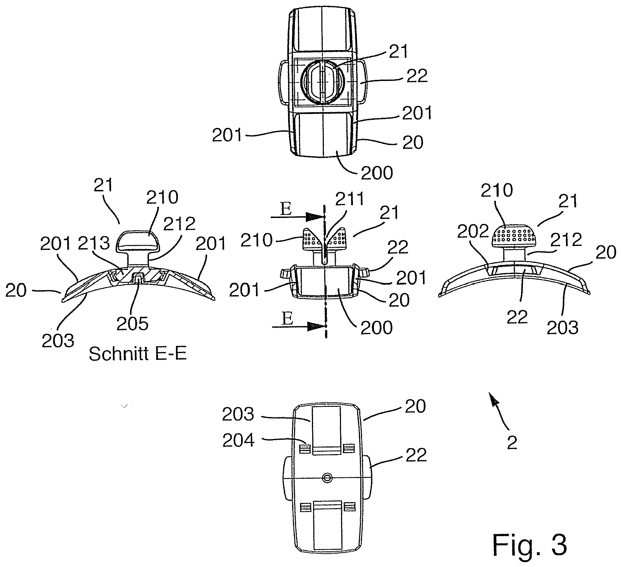

Wie aus Fig. 2 deutlicher ersichtlich, ist auf der dem körperseitigen Teil der Schlaufe bzw. des Abschnürbandes 1 zugekehrten Unterseite 203 des Unterteils 20 ein Befestigungsteil 24 angeordnet, welches zumindest eine rutschfeste, vorteilhaft griffige Auflage der Spanneinheit 2 auf dem betreffenden Abschnitt des Abschnürbands 1 ergibt, womit die Spanneinheit 2 beim Aufsetzen auf das Abnschnürbands 1 eine vereinfachte Handhabung und gute Funktion beim Spannen der Schlaufe ergibt. Vorteilhaft kann das Befestigungsteil 24 als Klettverschlussteil ausgebildet sein, das mit dem als erstes Verschlussteil ausgebildeten Abschnürband 1 zusammenwirkt, sodass die Spanneinheit 2 mit deren Unterteil 20 auf dem Abschnürband 1 beim Gebrauch fixiert wird, von diesem aber leicht nach Gebrauch entfernt werden kann. Fig. 2 lässt ebenfalls erkennen, dass der dem zweiten Verschlussteil 30 zugekehrte Abschnitt des Abschnürbands 1 durch den Spannteil 21 geführt ist. Hierfür weist das Spannteil 21 auf seiner von dem Unterteil 20 abgekehrten Seite einen Spannkopf 210 mit einem Schlitz 211 zum Durchführen des Abschnürbandes 1 auf, wie aus Fig. 3 näher ersichtlich.

Fig. 3 zeigt weitere Einzelheiten der Spanneinheit 2 in verschiedenen Ansichten. In der mittleren Darstellung ist die Spanneinheit 2 von vorne (in Führungsrichtung des Abschnürbandes 1) gezeigt. Auf dem Unterteil 20 ist das Spannteil 21 mit dem Spannkopf 210 und dem darin eingebrachten Schlitz 211 angeordnet, der sich bis in einen unterhalb des Spannkopfes 210 anschließenden Hals 212 erstreckt und sich zur Oberseite hin abgeschrägte Flanken zur einfachen Einführung des flexiblen Abschnürbandes 1 öffnet. In dieser Darstellung ist auch eine Bandauflage 200 des Unterteils 20 mit seitlichen Begrenzungen für eine einfache Bandführung zur erkennen. Seitlich an dem Unterteil 20 stehen in Querrichtung zu dem Unterteil 20 verstellbare Freigabemittel 22 vor, die zur Aufhebung eines arretierten Spannzustandes der Schlaufe dienen. Das Spannteil 21 mit dem Spannkopf 210 und dem darin eingebrachten Schlitz 211 sind auch in der Draufsicht (obere Darstellung) zu erkennen, in der auch die seitlich vorstehenden Freigabemittel 222 zu sehen sind. Wie des Weiteren in der seitlichen Ansicht (rechte Darstellung) und dem Längsschnitt (linke Darstellung) zu erkennen, ist das Spannteil 21 mit einem Spannfuß 213 versehen, welcher in einer Vertiefung 202 des Unterteils 20 eingesetzt ist. In der Vertiefung 202 befindet sich ein zapfenartig nach oben vorstehendes Drehlager 205, auf das der Spannfuß 213 mit einer angepassten Vertiefung drehbar eingesetzt ist, sodass ein Drehmechanismus zum Drehen des Spannteils 21 relativ zu dem Unterteil 21 gebildet wird. Somit kann das Spannteil 21 mit dem durch den Schlitz 211 geführten Bandabschnitt des Abschnürbands 1 gedreht und der Spannzustand der Schlaufe geändert werden.

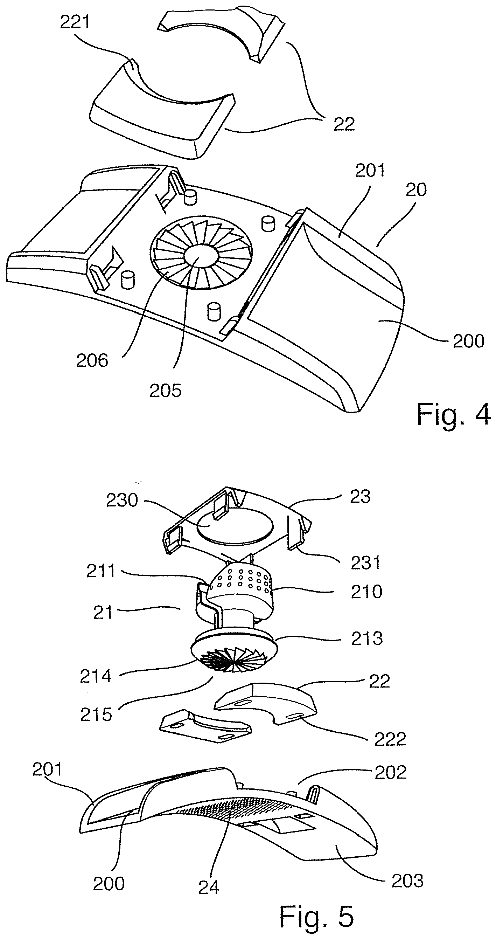

In der Ansicht von unten (untere Darstellung der Fig. 3) ist die Unterseite 203 des Unterteils 20 zu erkennen, in der vorliegend vier Fixierabschnitte 204 eingebracht sind, die zum Halten des Spannteils 21 und der Freigabemittel 229 mittels eines Fixierteils 23 (vgl. Fig.5) vorgesehen sind, welches bei aufgelegten Freigabemitteln 22 und eingesetztem Spannteil 21 von oben auf das Unterteil 20 aufsetzbar und mittels Fixiergegenabschnitten 231 an den Fixierabschnitten 204 festlegbar ist, vorliegend durch eine Rastverbindung. Jedoch sind auch andere Verbindungen, wie z. B. Schraubverbindungen zur Festlegung des Fixierteils 23 an dem Unterteil 20 mit dem Spannteil 21 und den Freigabemitteln 22 denkbar.

Fig. 4 zeigt das Unterteil 20 sowie die davon abgehobenen Freigabemittel 22 in perspektivischer Ansicht von oben. Dabei sind auch zum Zentrum hin gerichtete Abschrägungen 221 an den Freigabemitteln 22 ersichtlich, die zum Zusammenwirken mit in Fig. 5 gezeigten Gegenschrägen 214 an dem Spannfuß zum Freigeben eines Spannzustandes der verriegelten Schlaufe dienen.

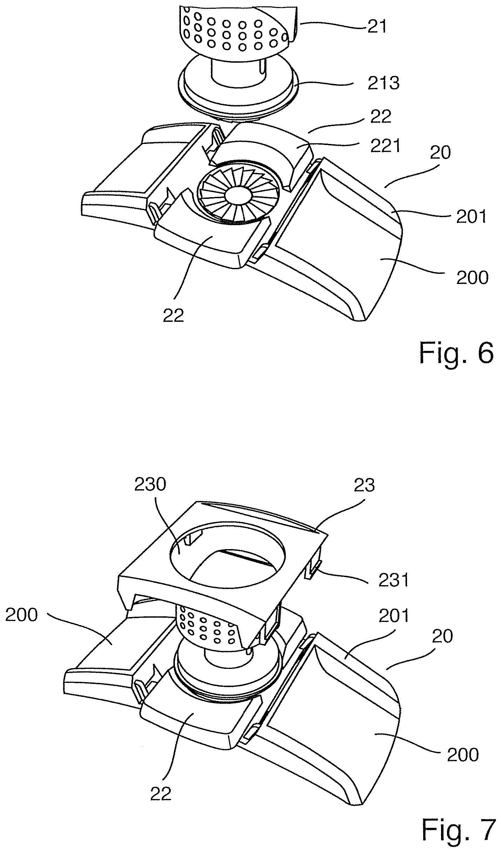

Wie Fig. 5 im Zusammenhang mit Fig. 4 und auch Fig. 6 erkennen lässt, ist einerseits im mittleren Bereich des Unterteils 20, und zwar in der Vertiefung 202 um das Drehlager 205, zum Bilden eines Arretierabschnitts 206 eine kreisringförmig umlaufende Zahnung ausgebildet, welche in einer Drehrichtung flache Zahnflanken und entgegen dieser Drehrichtung steile, beispielsweise rechtwinklig zur Unterseite stehende Zahnflanken aufweist. Wie Fig. 5 zeigt, ist das Spannteil 21 auf seiner dem Arretierabschnitt 206 zugekehrten Unterseite mit einem zu diesem komplementären Arretiergegenabschnitt 215 versehen, welcher eine mit der Zahnung des Arretierabschnitts 206 in Drehrichtung gleitend zusammenwirkende und entgegen dieser Drehrichtung blockierend zusammenwirkende Zahnung mit entsprechenden flachen und steilen Zahnflanken ringförmig um die Drehlagerung mit dem Drehlager 205 aufweist.

Das durch den Schlitz 211 geführte Abschnürband 1 drückt mit seiner Spannung das Spannteil 21 mit dessen Arretiergegenabschnitt 215 in den Arretierabschnitt 206 des Unterteils 20 und kann in Drehrichtung in Folge der aufeinandergleitenden Zahnflanken verstellt werden, wobei sich der Spannungszustand der Schlaufe erhöht. Mittels der entgegen der Drehrichtung wirkenden steilen Zahnflanken des Arretierabschnitts 206 und Arretiergegenabschnitts 215 wird ein Drehen in Gegenrichtung verhindert und der gewählte Spannungszustand der Schlaufe aufrechterhalten, wobei das gespannte Abschnürband 1 im Schlaufenbereich das Spannteil 21 mit seinem Arretiergegenabschnitt 215 in dem Arretierabschnitt 206 hält.

Ist die Spannung zu stark eingestellt, kann dieser Spannungszustand durch Anheben des Spannteils 21 unter Ziehen an dem Spannkopf 210 nach oben (von der Oberfläche des Körperteils weg) gelockert und damit in einen Spannungszustand mit geringerer Spannkraft zurückgestellt werden, z. B. um eine oder mehrere Zahnabstände, wo das Spannteil 21 mit seinem Arretiergegenabschnitt 215 in entsprechender Drehstellung beim Loslassen des Spannkopfes 21 in dem Arretierabschnitt 206 verrastet. Unterstütztend bei einer solchen Nachjustierung des Spannungszustandes kann eine gewisse Eigenelastizität des Abschnürbandes 1 wirken.

Insbesondere zum vollständigen Aufheben des Spannungszustandes werden die seitlich vorstehenden Freigabemittel 22 mit ihren Abschrägungen 221 nach innen unter die Gegenschräge 214 an der Unterseite des Spannteils 21 geschoben, wodurch das Spannteil 21 entgegen der Spannwirkung des Abschnürbands 1 aus dem Arretierabschnitt 206 herausgehoben wird und die Arretierung im Bereich der blockierenden Zahnflanken aufgehoben wird. Somit wird der Spannzustand der Schlaufe gelöst. Zum Öffnen der Schlaufe und Abnehmen der Abschnürvorrichtung kann dann die Verschlussvorrichtung geöffnet werden. Die Spanneinheit 2 kann von dem Abschnürband 1 abgenommen und das Abschnürband 1 kann bei Bedarf entsorgt werden.

Wie Fig. 5 erkennen lässt, sind die Freigabemittel 22 an ihrer Unterseite mit in Querrichtung länglichen Führungsstrukturen 222, vorliegend Vertiefungen, versehen, welche mit in der Vertiefung 202 des Unterteils 20 angeordneten komplementären Führungsgegenelementen, vorliegend also kleinen Vorsprüngen, zusammenwirken, um die Freigabemittel 22 in betreffendem Ausmaß zum Arretieren des Spannzustandes nach außen zu verschieben und zum Aufheben des Spannzustandes bzw. der Arretierung nach innen zu verschieben. Die Abschrägungen 221 und Gegenschrägen 214 sind dabei so aufeinander abgestimmt, dass die Freigabemittel durch die auf den Spannkopf ausgeübte Andruckkraft des Abschnürbandes 1 selbsttätig nach außen bewegt werden. Das Fixierteil 23 weist zum Aufsetzen auf das Unterteil 20 und Durchtreten des Spannkopfes 210 einen Durchbruch 230 auf, welcher einen geringeren Durchmesser besitzt als der Spannfuß 213, sodass das Spannteil 21 mittels des Fixierteils 23 auf dem Unterteil 20 zusammen mit den unter dem Spannfuß 213 angeordneten Freigabemitteln 22 sicher gehalten wird. Dabei besteht aber zwischen der Oberseite des Spannfußes 213 und der Unterseite des Fixierteils 23 so viel Spielraum, dass das Spannteil 21 mittels der Freigabemittel 22 aus der Arretierstellung angehoben werden kann, wobei die Anhebung parallel zur Drehachse bzw. senkrecht zu einer gedachten Tangentialebene gerichtet ist, welche das Unterteil 20 in einer gedachten querverlaufenden Mittellinie berührt.

Denkbar wäre auch eine Spanneinheit 2 mit einem Spannmechanismus, welcher beispielsweise eine quer zu dem Unterteil 20 in der Tangentialebene liegende Drehachse für einen Spannmechanismus aufweist. Als Arretiermechanismus wäre auch ein Klemmmechanismus denkbar.

## Patentansprüche

1. Abschnürvorrichtung für Körperteile, insbesondere Venenstauer, mit einem um ein Körperteil legbaren flexiblen Abschnürband (1) mit einer Verschlussvorrichtung, mittels deren das Abschnürband (1) in seinem als geschlossene Schlaufe um das Körperteil gelegten Zustand verriegelbar ist, wobei
- eine von der Verschlussvorrichtung separate Spanneinheit (2) vorhanden ist, durch die ein innerhalb der Schlaufe liegender Abschnitt des Abschnürbands (1) führbar ist und mittels deren die um das Körperteil gelegte, mittels der Verschlussvorrichtung verriegelte Schlaufe spannbar und in einem gewünschten Spannzustand mittels eines Arretiermechanismus arretierbar ist,
- die Spanneinheit (2) ein auf einem Abschnitt des Abschnürbandes (1) im Bereich der Schlaufe mit einer rutschfesten Unterseite (203) aufsetzbares Unterteil (20) aufweist,
- die Spanneinheit (2) ein auf dem Unterteil (20) drehbar gelagertes Spannteil (21) aufweist, welches mit einem Schlitz (211) zum Durchführen des Abschnürbandes (1) versehen ist, und das Abschnürband (1) durch Drehen des Spannteils (21) relativ zu dem Unterteil (20) spannbar ist, wobei das Spannteil (21) gegen ein Drehen entgegen der Spannrichtung mittels eines Arretiermechanismus blockiert ist,
- der Arretiermechanismus einen an dem Spannteil (21) angeordneten Arretierabschnitt (206) und einen diesem zugekehrten und mit diesem zusammenwirkenden, an dem Unterteil (20) angeordneten Arretiergegenabschnitt (215) aufweist, und
das Spannteil (21) um eine bezüglich einer Tangentialebene des Körperteils in einer Berührungslinie aufrecht stehende Drehachse drehbar an dem Unterteil (20) in einem Drehlager (205) gelagert ist.

2. Abschnürvorrichtung nach Anspruch 1,
wobei die Verschlussvorrichtung als Klettverschluss, Druckknopfverschluss oder Hakenverschluss ausgebildet ist.

3. Abschnürband nach Anspruch 1 oder 2, wobei die Verschlussvorrichtung als Klettverschluss mit zwei verriegelnd ineinandergreifenden Verschlussteilen ausgebildet ist.

4. Abschnürvorrichtung nach Anspruch 3,
wobei das Abschnürband (1) mit den ersten Verschlussteil bildenden Schlaufen einerseits und in diese zum Herstellen des Verschlusses verriegelnd eingreifenden, den zweiten Verschlussteil (30) bildenden Hakenelementen andererseits versehen ist.

5. Abschnürvorrichtung nach einem der Ansprüche 1 bis 4,
wobei der erste Verschlussteil aus dem Bandmaterial selbst und der zweite Verschlussteil (30) aus einem auf dem Bandmaterial befestigten Materialabschnitt gebildet ist.

6. Abschnürvorrichtung nach einem der vorhergehenden Ansprüche,
wobei der Arretierabschnitt (206) und der Arretiergegenabschnitt (215) als um die Drehachse angeordnete komplementäre Zahnstrukturen mit in Spannrichtung flachen, aufeinandergleitenden Zahnflanken und entgegen der Spannrichtung steilen, blockierenden Zahnflanken versehen sind.

7. Abschnürvorrichtung nach einem der vorhergehenden Ansprüche,
wobei eine Freigabevorrichtung vorhanden ist, mittels deren der arretierte Spannzustand aufhebbar ist.

8. Abschnürvorrichtung nach Anspruch 7,
wobei die Freigabevorrichtung zum Freigeben des arretierten Spannzustands durch Anheben des Spannteils (21) entgegen einer dieses mit seinem Arretiergegenabschnitt (215) in den Arretierabschnitt (206) zwingenden Andruckkraft ausgebildet ist.

9. Abschnürvorrichtung nach Anspruch 8,
wobei die Andruckkraft mittels des Abschnürbands (1) in dessen Spannzustand erzeugt ist.

10. Abschnürvorrichtung nach Anspruch 8 oder 9,
wobei die Freigabevorrichtung in den Bereich zwischen dem Spannteil (21) und dem Unterteil (20) einschiebbare, mit Abschrägungen (221) versehene verschiebbare Freigabemittel (22) aufweist.

11. Abschnürvorrichtung nach einem der vorhergehenden Ansprüche,
wobei das Spannteil (21) mittels eines Fixierteils (23) unverlierbar an dem Unterteil (20) gehalten ist.

12. Abschnürvorrichtung nach einem der vorhergehenden Ansprüche,
wobei die Unterseite des Unterteils (20) mit einem Befestigungsteil (24), insbesondere Klettverschlussteil, versehen ist, mittels dessen die Spanneinheit (2) auf dem Abschnürband (1) abnehmbar fixierbar ist.

## Claims

1. Constriction device for body parts, in particular a tourniquet, comprising a flexible constriction band (1) which can be placed around a body part and has a closure device, by means of which the constriction band (1) can be locked in the state thereof in which it is placed around the body part as a closed loop, wherein
- a tightening unit (2) is provided which is separate from the closure device, through which tightening unit a portion of the constriction band (1) located within the loop can be guided, and by means of which the loop which is placed around the body part and locked by means of the closure device can be tightened and can be locked in a desired tightened state by means of a locking mechanism,
- the tightening unit (2) has a lower part (20) a non-slip lower face (203) of which can be placed on a portion of the constriction band (1) in the region of the loop,
- the tightening unit (2) has a tightening part (21) which is rotatably mounted on the lower part (20) and is provided with a slot (211) for passing the constriction band (1) through, and the constriction band (1) can be tightened relative to the lower part (20) by rotating the tightening part (21), wherein the tightening part (21) is blocked against rotating counter to the tightening direction by means of a locking mechanism,
- the locking mechanism has a locking portion (206) which arranged on the tightening part (21), and a locking counter-portion (215) which is arranged on the lower part (20) and faces and interacts with said locking portion, and the tightening part (21) is mounted on the lower part (20) in a pivot bearing (205) so as to be rotatable about an axis of rotation which is upright at a tangent with respect to a tangential plane of the body part.

2. Constriction device according to claim 1, wherein the closure device is designed as a hook and loop fastener, snap fastener or hook fastener.

3. Constriction band according to either claim 1 or claim 2, wherein the closure device is designed as a hook and loop fastener having two interlocking closure parts.

4. Constriction device according to claim 3, wherein the constriction band (1) is provided on one side with loops which form the first closure part, and on the other side is provided with hook elements which engage in said loops in a locking manner in order to produce the closure, and which form the second closure part (30).

5. Constriction device according to any of claims 1 to 4, wherein the first closure part is formed from the band material itself, and the second closure part (30) is formed from a portion of material that is fastened to the band material.

6. Constriction device according to any of the preceding claims, wherein the locking portion (206) and the locking counter-portion (215) are provided as complementary tooth structures arranged about the axis of rotation, which tooth structures have tooth flanks which are flat in the tightening direction and slide on one another, and blocking tooth flanks which are steep counter to the tightening direction.

7. Constriction device according to any of the preceding claims, wherein a release device is provided, by means of which the locked tightened state can be ended.

8. Constriction device according to claim 7, wherein the release device is designed to release the locked tightened state by lifting the tightening part (21) counter to a pressing force which forces the locking counter-portion (215) of said tightening part into the locking portion (206).

9. Constriction device according to claim 8, wherein the pressing force is generated by means of the constriction band (1) in the tightened state thereof.

10. Constriction device according to either claim 8 or claim 9, wherein the release device has slidable release means (22) which can be slid into the region between the tightening part (21) and the lower part (20) and are provided with bevels (221).

11. Constriction device according to any of the preceding claims, wherein the tightening part (21) is captively secured on the lower part (20) by means of a fixing part (23).

12. Constriction device according to any of the preceding claims, wherein the lower face of the lower part (20) is provided with a fastening part (24), in particular a hook and loop fastener part, by means of which the tightening unit (2) can be removably fixed to the constriction band (1).

## Revendications

1. Dispositif de ligature destiné à des parties du corps, en particulier garrot, comportant une bande de ligature (1) souple pouvant être placée autour d'une partie du corps et comportant un dispositif de fermeture au moyen duquel la bande de ligature (1) peut être verrouillée dans son état placé autour de la partie du corps en tant que boucle fermée,
- une unité de serrage (2) séparée du dispositif de fermeture étant présente, par laquelle une section de la bande de ligature (1) située à l'intérieur de la boucle peut être guidée et au moyen de laquelle la boucle placée autour de la partie du corps et verrouillée au moyen du dispositif de fermeture peut être serrée et bloquée dans un état de serrage souhaité au moyen d'un mécanisme de blocage,
- l'unité de serrage (2) présentant une partie inférieure (20) pouvant être installée sur une section de la bande de ligature (1) dans la zone de la boucle au moyen d'une face inférieure antidérapante (203),
- l'unité de serrage (2) présentant une partie de serrage (21) qui est montée rotative sur la partie inférieure (20), la partie de serrage étant pourvue d'une fente (211) permettant d'y faire passer la bande de ligature (1), et la bande de ligature (1) pouvant être serrée par la rotation de la partie de serrage (21) relativement à la partie inférieure (20), une rotation de la partie de serrage (21) dans le sens inverse de la direction de serrage étant empêchée au moyen d'un mécanisme de blocage,
- le mécanisme de blocage présentant une section de blocage (206) disposée sur la partie de serrage (21) et, faisant face à celle-ci, une contre-section de blocage (215) disposée sur la partie inférieure (20) et coopérant avec la section de blocage, et
la partie de serrage (21) étant montée rotative sur la partie inférieure (20) dans un palier rotatif (205) autour d'un axe de rotation vertical dans une ligne de contact par rapport à un plan tangentiel de la partie du corps.

2. Dispositif de ligature selon la revendication 1,
dans lequel le dispositif de fermeture est conçu comme une fermeture velcro, une fermeture à bouton-pression ou une fermeture à crochet.

3. Bande de ligature selon la revendication 1 ou 2,
dans laquelle le dispositif de fermeture est conçu comme une fermeture velcro comportant deux parties de fermeture s'emboîtant l'une dans l'autre par verrouillage.

4. Dispositif de ligature selon la revendication 3,
dans lequel la bande de ligature (1) est pourvue de boucles formant la première partie de fermeture d'une part et d'éléments de crochet s'emboîtant avec celles-ci par verrouillage afin de produire la fermeture et formant la seconde partie de fermeture (30) d'autre part.

5. Dispositif de ligature selon l'une des revendications 1 à 4,
dans lequel la première partie de fermeture est formée à partir du matériau de bande lui-même et la seconde partie de fermeture (30) est formée à partir d'une section de matériau fixée au matériau de bande.

6. Dispositif de ligature selon l'une des revendications précédentes,
dans lequel la section de blocage (206) et la contre-section de blocage (215) sont pourvues de flancs dentaires plats glissant les uns sur les autres dans la direction de serrage et de flancs dentaires raides faisant blocage dans le sens inverse de la direction de serrage, en tant que structures dentaires complémentaires disposées autour de l'axe de rotation.

7. Dispositif de ligature selon l'une des revendications précédentes,
dans lequel un dispositif de libération est présent, au moyen duquel l'état de serrage bloqué peut être débloqué.

8. Dispositif de ligature selon la revendication 7,
dans lequel le dispositif de libération est conçu pour libérer l'état de serrage bloqué en soulevant la partie de serrage (21) à l'encontre d'une force de pression qui force cette dernière dans la section de blocage (206) au moyen de sa contre-section de blocage (215).

9. Dispositif de ligature selon la revendication 8,
dans lequel la force de pression est générée au moyen de la bande de ligature (1) dans l'état de serrage de cette dernière.

10. Dispositif de ligature selon la revendication 8 ou 9,
dans lequel le dispositif de libération présente des moyens de libération coulissants (22) comportant des biseaux (221) et pouvant être insérés dans la zone entre la partie de serrage (21) et la partie inférieure (20).

11. Dispositif de ligature selon l'une des revendications précédentes,
dans lequel la partie de serrage (21) est maintenue imperdable sur la partie inférieure (20) au moyen d'une partie d'immobilisation (23).

12. Dispositif de ligature selon l'une des revendications précédentes,
dans lequel la face inférieure de la partie inférieure (20) est pourvue d'une partie de fixation (24), en particulier d'une partie de fermeture velcro, au moyen de laquelle l'unité de serrage (2) peut être immobilisée de manière amovible sur la bande de ligature (1).
